# EUROPEAN PATENT SPECIFICATION

(11) **EP 2 081 597 B1**
(45) Date of publication and mention of the grant of the patent: **04.07.2012**
(21) Application number: 07821451.7
(22) Date of filing: 17.10.2007
(51) Int. Cl.: A61K 45/06, A23L 1/29, A61P 3/02, A23L 1/302, A23L 1/303, A23L 1/304, A23L 1/305

(54) **LONG-TERM ENTERAL FEED FOR MAINTENANCE**
LANGFRISTIGE ENTERALE ERHALTUNGSERNÄHRUNG
ALIMENTATION PAR VOIE ENTERALE POUR ENTRETIEN A LONG TERM

(30) Priority: 19.10.2006 US 862168 P
(43) Date of publication of application: 29.07.2009
(73) Proprietor: Nestec S.A., 1800 Vevey (CH)
(72) Inventor: LE-HENAND, Hervé, 78100 Saint Germain En Laye (FR); MURBACH, François, 01220 Divonne (FR); JEDWAB, Michael, CH-1012 Lausanne (CH); ROESSLE, Claudia, CH-1110 Morges (CH); CYNOBER, Luc, 75270 Paris (FR); BURDE, Alain, 51100 Reims (FR); LOCHS, Herbert, 100117 Berlin (DE)
(74) Representative: Lock, Graham James
(86) International application number: PCT/EP2007/061086
(87) International publication number: WO 2008/046857

(56) References cited:
- EP-A- 1 010 374
- EP-A- 1 797 891
- US-A- 5 472 952
- US-A- 5 686 429
- US-A- 5 792 754
- US-A- 5 985 339
- US-A1- 2004 067 224

## Description

### BACKGROUND

The present application relates to nutrition. More specifically, the present invention relates to clinical nutrition.

Due to a variety of diseases, insults, and complications, patients may not be able to obtain the necessary nutrition by ingesting food through the mouth, e.g., eating food. Therefore, it has been known to provide clinical nutrition either enterally or parenterally. A variety of different formulations have been developed to provide such clinical nutrition.

Even with respect to typical enteral nutritional products, these products are designed for short-term use, typically 10 to 24 days. In this regard, the products usually provide the essential nutritional components to provide necessary nutrition to patients having acute pathologies during their hospital stays. Although these products are suitable for such short term use, they have not necessarily been designed for long-term feeding of patients. With advances in medicine resulting in increased life expectancy and better disease treatments, a number of individuals could benefit from products designed to provide long-term enteral nutrition.
EP 1797891 by Ajinomoto Co., Inc. relates to a total enteral nutritional composition. The document discloses a total enteral nutritional composition containing sugars, proteins, fats, minerals and vitamins, especially zinc and copper. The composition is disclosed to supply total required nutrition to individuals unable to obtain sufficient nutrition from eating, such as the elderly and in-patients. It is disclosed that the composition has a notable effect toward improving serum levels of the nutritional index albumin and improving the condition of bedsores. In addition, the document discloses testing of the disclosed composition wherein the administration period was eight weeks.

### SUMMARY

The present invention provides methods for providing long-term tube-fed nutrition. More specifically, the present invention provides methods for providing long-term tube-fed nutrition to a normo-metabolic patient who is unable to eat a normal diet.

To this end, methods according to the invention are defined in the appended claims.

In an embodiment of the method, the source of protein provides 10 to 18% by caloric content of the product. The protein source can be selected from the group consisting of casein, whey, and soy. Moreover, the protein can be intact or partially hydrolyzed. With respect to the carbohydrate source, it can provide 40 to 65% by caloric content of the product. The source of lipids can provide 25 to 40% by caloric content of the product, with saturated fatty acids of not greater than 1.1 g/100 kcal, the composition contains between 0.3 and 1.1 g linoleic acid per 100 kcal, the composition contains at least 0.06 g linolenic acid per 100 kcal, and the n6:n3 ratio is between 2 and 7. The product can also contain a source of dietary fiber that provides at least 10 g/l. The fiber can comprise insoluble fibers and soluble fibers. For example, the insoluble fiber can comprise at least 25% of the fiber source. The fiber can comprise soy polysaccharides and pea outer fibers.

If desired, the composition can comprise a prebiotic. In an embodiment, the prebiotic can be inulin. In another embodiment, the product has a density of 0.8 to 1.4 kcal/ml.

An advantage of the present invention is to provide improved enteral nutrition products.

Another advantage of the present invention is to provide enteral nutrition products targeted to long-term use.

Furthermore, an advantage of the present invention is to provide compositions for providing long-term nutrition to a patient requiring same.

Additionally, an advantage of the present invention is to provide methods for providing long-term nutrition to a patient requiring same.

Additional features and advantages are described herein, and will be apparent from, the following Detailed Description.

### DETAILED DESCRIPTION

The present invention relates to clinical nutrition. More specifically, the present invention relates to providing long-term tube-fed nutrition to patients requiring same. As used herein, the term "long-term" means greater than one month (30 days). As used herein, the term "tube-fed" means to provide a product to a patient through a feed tube that is received within a portion of the digestive tract of a patient, for example, a percutaneous endoscopic gastrostomy or nasogastric feed tube.

The long-term tube-fed nutrition product is designed for maintenance patients. As used herein, "maintenance patient" refers to an adult patient under the age of sixty-five who cannot receive nutrition through a normal diet but who is normo-metabolic (i.e. not suffering from a metabolic disorder). Such a patient may previously have undergone surgery for a cancer of the head or neck leaving an incomplete digestive tract or an inability to swallow, may have received an injury to the neck leaving him or her unable to swallow or may be unable to swallow as a result of neurological damage caused by a stroke for example. As used herein, the term "normal diet" means to receive at least substantially all nutrition by eating, i.e., using one's mouth, without the use of any feed tube or parenteral feed.

The present invention provides methods as well as products that are optimized and/or improved for long-term use, especially to provide complete nutrition to maintenance patients, as compared to standard enteral nutrition products. As used herein, the term "standard enteral nutrition product" refers to products that are not specifically advertised or promoted for long-term use. A variety of such products are available, for example, from Nestle Clinical Nutrition, Abbott, Novartis, Numico, and Fresenius. In an embodiment, these product are provided to the patient outside of a hospital setting. For example, the products can be provided in a nursing home, out care patient center, or even the home of the patient. Preferably, the nutrition products are housed in a plastic bag. A variety of such bags are known, for example, 500 ml, 1000 ml, and 1500 ml bags are known in the art. It should be noted, however, that any suitable container can be used to house the nutrition product. Typically, the product is administered so that the patient receives 1500 ml per day, although those skilled in the art will appreciate that variations to the amount of product administered are possible.

Because the long-term enteral nutrition formulation of the present invention is provided for maintenance, it is not directed to any specific, qualitative, or quantitative complement. Patients are typically stable, normo-metabolic, healthy patients except for the fact that they require enteral nutrition in order to receive necessary nutritional requirements. Thus, these patients can suffer from a variety of disorders including swallowing disorders of a variety of etiologies, particularly surgical consequences of ear/nose/throat cancer, and patients suffering from a cerebral vascular accident.

One of the goals of the formulation is to optimize metabolic status and stability in long-term enteral fed patients. By providing not only necessary macronutrients but also the micronutrients that contribute to, for example the antioxidant status, the formulation can maintain the metabolic status of the body in a comparable condition to a completely healthy individual of the same age eating a balanced diet. Thus, the present invention provides a method of improving the metabolic stability of long-term enteral fed patients.

Although the formula is designed to provide, in a preferred embodiment, necessary nutritional minerals, and vitamins to meet government requirements (defined below), there are some exceptions with respect to these recommendations. In this regard, preferably, excess calcium is utilized. In this regard, in an embodiment, preferably at least 33 percent more calcium is utilized. One of the reasons for this increase is that these patients have a reduced physical activity. In addition, excess vitamin D is preferably provided. In a preferred embodiment, at least 150 percent more vitamin D is provided than required by at least certain government requirements. Because of their reduced mobility, these patients are exposed to less sunlight and consequently have less endogenous synthesis of this vitamin. The maintenance of a satisfactory bone reserve is expected from this increased calcium and vitamin D intake. Also, in the formulation in an embodiment, the iron intake corresponds to the typical governmental requirements for females. These are usually considerably higher than that of a male. The idea is to avoid recurrence of an iron deficiency to which women are predisposed.

The protein source preferably provides 10 to 18 percent by caloric content of the product. Any high quality protein source or mixture thereof can be utilized. Examples include casein, whey, and soy protein. Proteins may be intact or partially hydrolyzed. Free amino acids may be added if desired. In an embodiment, a mixture is utilized of 50 percent caseinate and 50 percent soy. Preferably, the protein source is obtained through a mix of caseins and soy proteins allowing a balanced intake of amino acids.

The carbohydrate source preferably comprises 40 to 65 percent by caloric content of the product. Any carbohydrate or mixture of carbohydrates can be utilized. Examples include starches, maltodextrins, sucrose, and mixtures thereof. In an embodiment, 100 percent maltodextrins are used.

Preferably, the lipids comprise 25 to 40 percent by caloric content of the product. Any suitable mixture of dietary lipids can be provided including saturated fatty acids (SFA), monounsaturated fatty acids (MUFA), polyunsaturated fatty acids (PUFA), and medium-chain triglycerides (MCT). Preferably, saturated fatty acids are present in an amount less than 1.1 g/100 kcal. Preferably, the composition contains between 0.3 and 1.1 g linoleic acid (or higher derivative thereof) per 100 kcal. The composition may contain at least 0.06 g/linolenic acid, or higher derivative thereof, per 100 kcal. The n6:n3 ratio is preferably 2 to 7.

Preferably, the composition has an energy density of 0.80 to 1.4 kcal/ml.

Fiber intake is preferably high in the formula of the present invention. Constipation presents itself frequently in this patient population. Preferably, the fiber composition comprises at least 10 g/l. Any suitable fiber or mix of fibers can be used. Examples of insoluble fibers are soy polysaccharides, pea outer fiber. Examples of soluble fibers are acacia gum, pectin, inulin, and guar gum. Generally, a mixture of soluble and insoluble fibers is preferred. In addition, prebiotic fibers may be included. A prebiotic is defined as a non-digestible food ingredient that beneficially affects the host by selectively stimulating the growth and/or activity of one or a limited number of bacteria in the colon and thus improves host health. Examples of prebiotic fibers include acacia gum and fructo-oligosaccharides such as inulin and hydrolysed inulin. In an embodiment, a mixture of 50 percent pea outer fiber, 37 percent pea inner fiber and 13 percent prebiotic fiber (inulin and hydrolysed inulin) at 16.7 g/l is used. This corresponds to a mixture of 66% insoluble fiber and 34% soluble fiber (including the prebiotic fiber).

The nutrition products are specifically designed, in an embodiment, so that they can provide complete long-term nutrition and attempt to provide the same macro and micro nutrients as would be ingested by a healthy person eating a balanced diet. Therefore, the formulas mimic, in an embodiment, what is referred to herein as the 5/8 a day. As used herein, the term "5/8 a day" refers to governmental guidelines to consumers to eat five to eight helpings of fruits and vegetable per day. Thus, in an embodiment, the products are designed so that, to the extent possible, they attempt to mimic a normal diet that is preferably ingested by individuals that do not require a tube-fed product by providing micronutrients and phytonutrients found in fruit and vegetables. In an embodiment, the present invention provides a method of designing long-term enteral nutrition products based on attempting to mimic the 5/8 a day. By providing such a nutrition product, the patient's antioxidant status can be maintained as well as metabolic status. A goal being to place these patients in a state comparable, to the extent possible, to that of a completely healthy individual of the same age eating a balanced diet.

Phytonutrients have been found to provide the following characteristics: antioxidant, anti-inflammatory, detoxification, cancer protective, prevention of atherosclerosis, alleviation of metabolic syndromes, and prevention of bone loss. To achieve the necessary phytonutrients, the compositions of the present invention can include one or more of carotenoids such as lycopene (tomato), B-carotene (carrot, spinach, tomato), lutein (spinach), B-cryptoxanthin, vitamins such as mixed tocopherols (oils and nuts), and vitamin C (orange); and polyphenols such as catechins (green tea).

Preferably, the products include the necessary nutritional components to provide complete nutrition to the patient on a long-term basis. In this regard, the products include, among other possible ingredients: protein, carbohydrate, fat, vitamins, and minerals. In an embodiment, the products substantially, if not completely, comply with at least certain governmental requirements. As used herein, "governmental requirements" means any recommendations from any one of the following governments: U.S., typically the USRDA; German, typically the German RDA; and French, typically the French RDA. In an embodiment, the nutrition product meets or exceeds at least one of the governmental requirements.

By way of example and not limitation, examples of the present invention will now be given.

### Example No. 1

| | | **Embodiment Maintenance per 1500 ml** | **Embodiment per per 100 ml** |
|---|---|---|---|
| **Calories** | Kcal | **1875** | **125** |
| **Protein** | g | **62** | **4.1** |
| Ca Caseinate | g | **31** | **2.06** |
| Soya | g | **31** | **2.06** |
| **Carbohydrates** | g | **252** | **16.8** |
| Maltodextrins | g | **237** | **15.8** |
| Carbohydrates from other sources | g | **15** | **1.0** |
| **Fiber** | g | **23** | **1.52** |
| | | | |
| Insoluble | % | **66** | **66** |
| Soluble | % | **34** | **34** |
| | | | |
| | | | |
| **Lipids** | g | **72** | **4.8** |
| SFA | g | **11** | **0.73** |
| MUFA | g | **43** | **2.9** |
| PUFA | g | **11** | **0.73** |
| linoleic acid (n-6) | g | **8.4** | **0.56** |
| α linolenic acid (n-3) | g | **1.6** | **0.11** |
| Ratio ω6/ω3 | | **5.2** | **5.2** |

| **Minerals and Trace Elements** | | | |
|---|---|---|---|
| Sodium | mg | **2400** | **160** |
| Potassium | mg | **2445** | **163** |
| Calcium | mg | **1290** | **86** |
| Phosphorous | mg | **855** | **57** |
| Magnesium | mg | **405** | **27** |
| Chloride | mg | **3225** | **215** |
| Iron | mg | **18** | **1.2** |
| Zinc | mg | **12** | **0.78** |
| Copper | mg | **2** | **0.13** |
| Fluoride | mg | **1.4** | **0.09** |
| Chromium | µg | **105** | **7.0** |
| Molybdenum | µg | **98** | **6.5** |
| Selenium | µg | **81** | **5.4** |
| Manganese | mg | **4.4** | **0.29** |
| Iodine | µg | **165** | **11** |

| **Vitamins** | | | |
|---|---|---|---|
| Vitamin A total | IU | **4500** | **300** |
| Vitamin D | µg | **20** | 1.3 |
| Vitamin E | IU | **48** | **3.2** |
| Vitamin K | µg | **105** | **7.0** |
| Vitamin C | mg | **180** | **12.0** |
| Vitamin B1 (Thiamin) | mg | **2.0** | **0.13** |
| Vitamin B2 (Riboflavin) | mg | **1.7** | **0.11** |
| Vitamin B3-PP (Niacin) | mg | **23** | **1.50** |
| Vitamin B5 (Pantothenic acid) | mg | **9.5** | **0.63** |
| Vitamin B6 (Pyridoxine) | mg | **2.3** | **0.15** |
| Vitamin B8 (Biotin) | µg | **57** | **3.8** |
| Vitamin B9 (Folic Acid) | µg | **450** | **30** |
| Vitamin B12 | µg | **5.7** | **0.38** |

| **Other** | | | |
|---|---|---|---|
| Choline | mg | **810** | **54** |
| Taurine | mg | **81** | **5.4** |
| Carnitine | mg | **150** | **10** |
| Beta-carotene (carrot) | mg | **3.8** | **0.25** |
| Lycopene (tomato) | mg | **5.9** | **0.39** |

### Example No. 2

| | | **Embodiment Maintenance per 1500 ml** | **RANGE for 100 kcal** | **Embodiment per 100 ml** |
|---|---|---|---|---|
| **Calories** | kcal | **1875** | 0.8-1.4kcal/ml | **125** |
| **Protein** | g | **62** | 10-18% of total energy content, intact or partially hydolysed | **4.1** |
| Ca Caseinate | g | **31** | | **2.06** |
| Soya | g | **31** | | **2.06** |
| **Carbohydrates** | g | **252** | 40-65% of total energy content | **16.8** |
| Maltodextrins | g | **237** | | **15.8** |
| Carbohydrates from other sources | g | **15** | | **1.0** |
| Fibers | g | **23** | >10g/litre | **1.5** |
| Insoluble | % | **66** | | **66** |
| Soluble | % | **34** | | **34** |
| **Lipids** | g | **72** | 25-40% of total energy content | **4.8** |
| SFA | g | **11** | saturated fats (not inc. MCT) <10% of total energy content: or <1.11g/100kcal | **0.73** |
| MUFA | g | **43** | | **2.9** |
| PUFA | g | **11** | | **0.73** |
| Linoleic acid (n-6) | g | **8.4** | 3-10% of total energy content linoleic acid or higher w6 derivatives or 0.33-1.11g/100kcal | **0.56** |
| α linolenic acid (n-3) | g | **1.6** | >0.6% of total energy content or >0.06g/100kcal | **0.11** |
| Ratio ω6/ω3 | | **5.2** | 2-7 | **5.2** |

| **Minerals and Trace Elements** | | | | |
|---|---|---|---|---|
| Sodium | mg | **2400** | 100-200 | **160** |
| Potassium | mg | **2445** | 25-250 | **163** |
| Calcium | mg | **1290** | At least 50 preferably 50-300 g | **86** |
| Phosphorus | mg | **855** | <150g preferably 40-80 | **57** |
| Magnesium | mg | **405** | At least 15 preferably 15-35 | **27** |
| Chlorides | mg | **3225** | At least 100 g preferably 150-250 | **215** |
| Iron | mg | **18** | 0.4-1.5 | **1.2** |
| Zinc | mg | **12** | 0.4-2.0 | **0.78** |
| Copper | mg | **2** | 0.08-0.4 | **0.13** |
| Fluoride | mg | **1.4** | <0.15 | **0.09** |
| Chromium | µg | **105** | 2-10 | **7.0** |
| Molybdenum | µg | **98** | 2-14 | **6.5** |
| Selenium | µg | **81** | 3-9 | **5.4** |
| Manganese | µg | **4.4** | 0.1-0.4 | **0.29** |
| Iodine | µg | **165** | 7-15 | **11** |

| **Vitamins** | | | | |
|---|---|---|---|---|
| Vitamin A Total | IU | **4500** | 100-500 inc. b-carotene | **300** |
| Vitamin D | µg | **20** | 0.5-2.5 | **1.3** |
| Vitamin E | IU | **48** | 2.2-6 | **3.2** |
| Vitamin K | µg | **105** | Greater than 4 preferably 6-15 | **7.0** |
| Vitamin C | mg | **180** | Greater than 4 | **12.0** |
| Vitamin B1 (Thiamin) | mg | **2.0** | Greater than 0.06 preferably 0.06-0.4 | **0.13** |
| Vitamin B2 (Riboflavin) | mg | **1.7** | Greater than 0.07 | **0.11** |
| Vitamin B3-PP (Niacin) | mg | **23** | 0.7-3.5 | **1.5** |
| Vitamin B5 (Panthothenic acid) | mg | **9.5** | 0.2-2.0 | **0.63** |
| Vitamin B6 (Pyridoxine) | mg | **2.3** | 0.1-0.7 | **0.15** |
| Vitamin B8 (Biotin) | µg | **57** | At least 1 | **3.8** |
| Vitamin B9 (Folic Acid) | µg | **450** | At least 12 | **30** |
| Vitamin B12 | µg | **5.7** | 0.1-1 | **0.38** |

| **Other** | | | | |
|---|---|---|---|---|
| Choline | mg | **810** | If present >30 | **54** |
| Taurine | mg | **81** | If present >4 | **5.4** |
| Carnitine | mg | **150** | If present >3 | **10** |
| Beta-carotene (carrot) | mg | **3.8** | >0.1 | **0.25** |
| Lycopene (tomato) | mg | **5.9** | >0.2 | **0.39** |

Pursuant to the methods of the claimed invention, by way of example, either the formulas of Examples 1 and 2 can be administered to a patient requiring nutrition who cannot eat a normal diet, at least once a day on a long-term basis for as long as necessary.

## Claims

1. A method for providing long-term tube-fed nutrition to an adult patient under the age of sixty-five who cannot receive nutrition through a normal diet but who is normometabolic comprising the steps of:
providing to the patient, at least once a day, for a long term, an enteral nutrition product through a tube comprising per 100 kcal of product:
a source of protein;
a source of carbohydrates;
a source of lipids;
sodium 100 to 200 mg;
potassium 25 to 250 mg;
calcium above 50 mg;
phosphorus less than 150 mg;
magnesium at least 15 mg;
chloride at least 100 mg;
iron 0.4 to 1.5 mg;
zinc 0.4 to 2.0 mg;
copper 0.08 to 0.4 mg;
fluoride 0 to 0.15 mg;
chromium 2.0 to 10.0 micrograms;
molybdenum 2.0 to 14.0 micrograms;
selenium 3.0 to 9.0 micrograms;
manganese 0.1 to 0.4 mg;
iodine 7.0 to 15.0 micrograms;
Vit A 100 to 500 IU;
Vit D 0.5 to 2.5 micrograms;
Vit E 1.5 to 4.0 mg;
Vit K greater than 4.0 micrograms;
Vit C greater than 4.0 mg;
Vit B1 greater than 0.06 mg;
Vit B2 greater than 0.07 mg;
Vit B3 0.7 to 3.5 mg;
Vit B5 0.2 to 2.0 mg;
Vit B6 0.1 to 0.7 mg;
Vit B8 at least 1.0 micrograms;
Vit B9 at least 12.0 micrograms;
Vit B12 0.1 to 1.0 micrograms;
at least 30 mg of choline;
at least 4.0 mg of taurine; and
at least 3.0 mg of carnitine.

2. The method of claim 1, wherein the enteral nutrition product further comprises:
lycopene;
B-cryptoxanthine; and
polyphenol, optionally wherein the polyphenols are selected from the group consisting of: catechin; isoflavons; and quercetin.

3. The method of claim 1 wherein the enteral nutrition product comprises:
saturated fatty acids of not greater than 1.1 g/100 kcal;
the composition contains between 0.3 and 1.1 g linoleic acid per 100 kcal;
the composition contains at least 0.06 g linolenic acid per 100 kcal; and
the n6:n3 ratio is between 2 and 7.

4. The method of claim 1 wherein:
the source of protein provides 10 to 18% by caloric content of the product; the source of carbohydrate provides 40 to 65% by caloric content of the product; the source of lipids provides 25 to 40% by caloric content of the product; and the product comprises a source of dietary fiber in an amount of at least 10 g/l.

5. The method of claim 4 wherein the fiber comprises insoluble fibers and soluble fibers.

6. The method of claim 5 wherein the insoluble fiber comprises at least 25% of the fiber source.

7. The method of claim 1 or 5 comprising a prebiotic, optionally wherein the prebiotic comprises inulin and/or acacia gum.

8. The method of claim 1 wherein the vitamin A is provided at least in part by beta-carotene.

9. The method of claim 4, wherein the fiber comprises soy polysaccharides and pea outer fibers.

10. The method of claim 1 wherein the product comprises a protein source selected from the group consisting of: casein, whey, and soy, optionally wherein the protein can be intact or partially hydrolyzed, or wherein the product has a density of 0.8 to 1.4 kcal/ml.

## Patentansprüche

1. Verfahren zur Bereitstellung von Langzeitsondennahrung an einen erwachsenen, unter 65-jährigen Patienten, welcher Nahrung nicht durch eine normale Ernährungsweise erhalten kann, der aber normo-metabolisch ist, wobei bei dem Verfahren:
dem Patienten mindestens einmal am Tag, über einen langen Zeitraum, ein Produkt zur enteralen Ernährung über eine Sonde bereitgestellt wird, das pro 100 kcal des Produkts enthält:
eine Proteinquelle;
eine Kohlenhydratquelle;
eine Lipidquelle;
Natrium 100 bis 200 mg;
Kalium 25 bis 250 mg;
Calcium mehr als 50 mg;
Phosphor weniger als 150 mg;
Magnesium mindestens 15 mg;
Chlorid mindestens 100 mg;
Eisen 0,4 bis 1,5 mg;
Zink 0,4 bis 2,0 mg;
Kupfer 0,08 bis 0,4 mg;
Fluorid 0 bis 0,15 mg;
Chrom 2,0 bis 10 Mikrogramm;
Molybdän 2,0 bis 14,0 Mikrogramm;
Selen 3,0 bis 9,0 Mikrogramm;
Mangan 0,1 bis 0,4 mg;
Jod 7,0 bis 15,0 Mikrogramm;
Vit A 100 bis 500 IE;
Vit D 0,5 bis 2,5 Mikrogramm;
Vit E 1,5 bis 4,0 mg;
Vit K mehr als 4,0 Mikrogramm;
Vit C mehr als 4,0 mg;
Vit B1 mehr als 0,06 mg;
Vit B2 mehr als 0,07 mg;
Vit B3 0,7 bis 3,5 mg;
Vit B5 0,2 bis 2,0 mg;
Vit B6 0,1 bis 0,7 mg;
Vit B8 mindestens 1,0 Mikrogramm;
Vit B9 mindestens 12,0 Mikrogramm;
Vit B12 0,1 bis 1,0 Mikrogramm;
mindestens 30 mg Cholin;
mindestens 4,0 mg Taurin; und
mindestens 3,0 mg Carnitin.

2. Verfahren nach Anspruch 1, wobei das Produkt zur enteralen Ernährung ferner enthält:
Lycopin;
B-Cryptoxanthin; und
Polyphenol, gegebenenfalls wobei die Polyphenole ausgewählt sind aus der Gruppe bestehend aus: Catechin; Isoflavonen; und Quercetin.

3. Verfahren nach Anspruch 1, wobei das Produkt zur enteralen Ernährung enthält:
gesättigte Fettsäuren nicht mehr als 1,1 g/ 100 kcal;
die Zusammensetzung zwischen 0,3 und 1,1g Linolsäure pro 100 kcal enthält;
die Zusammensetzung mindestens 0,06 Linolensäure pro 100 kcal enthält; und
das n6:n3-Verhältnis zwischen 2 und 7 ist.

4. Verfahren nach Anspruch 1, wobei:
die Proteinquelle 10 bis 18 % des Kaloriengehalts des Produktes bereitstellt; die Kohlenhydratquelle 40 bis 65 % des Kaloriengehalts des Produkts bereitstellt; die Lipidquelle 25 bis 40 % des Kaloriengehalts des Produkts bereitstellt; und das Produkt eine Quelle an Ballaststoffen in einer Menge von mindestens 10 g/l enthält.

5. Verfahren nach Anspruch 4, wobei die Ballaststoffe unlösliche Ballaststoffe und lösliche Ballaststoffe umfassen.

6. Verfahren nach Anspruch 5, wobei der unlösliche Ballaststoff mindestens 25 % der Ballaststoffquelle umfasst.

7. Verfahren nach Anspruch 1 oder 5, das ein Präbiotikum umfasst, gegebenenfalls wobei das Präbiotikum Inulin und/oder Gummiarabicum umfasst.

8. Verfahren nach Anspruch 1 oder 5, wobei das Vitamin A zumindest teilweise durch Betacarotin bereitgestellt wird.

9. Verfahren nach Anspruch 4, wobei der Ballaststoff Sojapolysaccharide und äußere Erbsenfasern umfasst.

10. Verfahren nach Anspruch 1, wobei das Produkt eine Proteinquelle enthält, die ausgewählt ist aus der Gruppe bestehend aus: Casein, Molke, und Soja, gegebenenfalls wobei das Protein intakt oder teilweise hydrolysiert sein kann, oder wobei das Produkt eine Dichte von 0,8 bis 1,4 kcal/ml hat.

## Revendications

1. Procédé pour fournir une nutrition par sonde de longue durée à un patient adulte âgé de moins de 65 ans qui ne peut pas recevoir de nourriture via une alimentation normale mais qui est normo-métabolique, comprenant les étapes consistant à :
fournir au patient, au moins une fois par jour, sur une longue durée, un produit de nutrition entérale à travers une sonde comprenant pour 100 kcal de produit :
une source de protéine;
une source de glucides;
une source de liquides;
sodium 100 à 200 mg;
potassium 25 à 250 mg;
calcium plus de 50 mg;
phosphore moins de 150 mg;
magnésium au moins 15 mg;
chlorure au moins 100 mg;
fer 0,4 à 1,5 mg;
zinc 0,4 à 2,0 mg;
cuivre 0,08 à 0,4 mg;
florure 0 à 0,15 mg;
chrome 2,0 à 10,0 microgrammes;
molybdène 2,0 à 14,0 microgrammes;
sélénium 3,0 à 9,0 microgrammes;
manganèse 0,1 à 0,4 mg;
iode 7,0 à 15,0 microgrammes;
vitamine A 100 à 500 UI;
vitamine D 0,5 à 2,5 microgrammes;
vitamine E 1,5 à 4,0 mg;
vitamine K plus de 4,0 microgrammes;
vitamine C plus de 4,0 mg;
vitamine B1 plus de 0,06 mg;
vitamine B2 plus de 0,07 mg;
vitamine B3 0,7 à 3,5 mg;
vitamine B5 0,2 à 2,0 mg;
vitamine B6 0,1 à 0,7 mg;
vitamine B8 au moins 1,0 microgrammes;
vitamine B9 au moins 12,0 microgrammes;
vitamine B12 0,1 à 1,0 microgrammes;
au moins 30 mg de choline;
au moins 4,0 mg de taurine; et
au moins 3,0 mg de carnitine.

2. Le procédé selon la revendication 1, dans lequel le produit de nutrition entérale comprend en outre :
lycopène;
B-cryptoxanthine; et
polyphénol, les polyphénols étant optionnellement choisis parmi le groupe constitué par : catéchine; isoflavones; et quercétine.

3. Le procédé selon la revendication 1, dans lequel le produit de nutrition entérale comprend :
des acides gras saturés ne dépassant pas 1,1 g/100 kcal;
la composition contient entre 0,3 et 1,1 g d'acide linoléique par 100 kcal;
la composition contient au moins 0,06 g d'acide linoléique par 100 kcal; et
le rapport n6:n3 est compris entre 2 et 7.

4. Le procédé selon la revendication 1, dans lequel :
la source de protéine fournit 10 à 18 % en teneur calorique du produit; la source de glucide fournit 40 à 65 % en teneur calorique du produit; la source de lipides fournit 25 à 40 % en teneur calorique du produit; et le produit comprend une source de fibre alimentaire dans une quantité d'au moins 10 g/l.

5. Le procédé selon la revendication 4, dans lequel la fibre comprend des fibres insolubles et des fibres solubles.

6. Le procédé selon la revendication 5, dans lequel la fibre insoluble comprend au moins 25 % de la source de fibre.

7. Le procédé selon la revendication 1 ou 5 comprenant un prébiotique, le prébiotique comprenant optionnellement de l'inuline et/ou de la gomme d'acacia.

8. Le procédé selon la revendication 1, dans lequel la vitamine A est fournie au moins en partie par du bêta-carotène.

9. Le procédé selon la revendication 4, dans lequel la fibre comprend des polysaccharides de soja et des fibres extérieures de pois.

10. Le procédé selon la revendication 1, dans lequel le produit comprend une source de protéine choisie parmi le groupe constitué de : caséine, lactosérum, et soja, optionnellement dans lequel la protéine peut être intacte ou partiellement hydrolysée, ou dans lequel le produit a une densité de 0,8 à 1,4 kcal/ml.
